# EUROPEAN PATENT SPECIFICATION

(11) **EP 1 625 116 B1**
(45) Date of publication and mention of the grant of the patent: **08.08.2007**
(21) Application number: 04729020.0
(22) Date of filing: 23.04.2004
(51) Int. Cl.: C07D 215/227, C07D 487/10

(54) **PROCESS FOR THE PREPARATION OF CARBOSTYRIL DERIVATIVES,SUCH AS ARIPIPRAZOLE AND ITS INTERMEDIATES**
VERFAHREN ZUR HESTELLUNG CARBOSTYRILDERIVATE WIE ARIPIPRAZOLE UND DEREN ZWISCHENVERBINDUNGEN
PROCEDE DE PREPARATION DE DERIVES DE CARBOSTYRYLE, TELS QUE L'ARIPIPRAZOLE ET SES INTERMEDIAIRES

(30) Priority: 08.05.2003 CA 2428237
(43) Date of publication of application: 15.02.2006
(73) Proprietor: Delmar Chemicals Inc., LaSalle, QC H8R 2B2 (CA)
(72) Inventor: SALAMA, Paul, Montreal, Quebec H2V 2P4 (CA); MEUNIER, Jean-Francois, Montreal, Quebec H2E 2Z1 (CA); LAFRENIERE, Julie, Saint-Bruno, Quebec J3V 5N8 (CA); WANG, Yuan, Brossard, Quebec J4X 2P6 (CA); LIU, Lu Wei, Brossard, Quebec J4X 2P6 (CA)
(74) Representative: Gervasi, Gemma
(86) International application number: PCT/CA2004/000605
(87) International publication number: WO 2004/099152

(56) References cited:
- EP-A- 0 367 141
- YASUHIRO TORISAWA: "progress in arylpiperazine synthesis by the catalytic amination reaction" BIOORGANIC & MEDICINAL CHEMISTRY ( 2002 ), 10(12), 4023-4027 CODEN: BMECEP; ISSN: 0968-0896, vol. 10, no. 12, 2002, pages 4023-4027, XP001182487 JAPAN
- OSHIRO YASUO ET AL: "Novel Antipsychotic Agents with Dopamine Autoreceptor Agonist Properties: Synthesis and Pharmacology of 7-[4-(4-Phenyl-1-piperaziny l)butoxy]-3,4- dihydro-2(1H)-quinolinone Derivatives" JOURNAL OF MEDICINAL CHEMISTRY, AMERICAN CHEMICAL SOCIETY. WASHINGTON, US, vol. 41, 1998, pages 658-667, XP002272484 ISSN: 0022-2623
- MORITA S ET AL: "Practical Application of the Palladium-catalyzed Amination in Phenylpiperazine Synthesis: An Efficient Synthesis of a Metabolite of the Antipsychotic Agent Aripiprazole" TETRAHEDRON, ELSEVIER SCIENCE PUBLISHERS, AMSTERDAM, NL, vol. 54, no. 19, 1 May 1998 (1998-05-01), pages 4811-4818, XP004117484 ISSN: 0040-4020

## Description

### FIELD OF THE INVENTION

The invention relates to novel processes and intermediates for preparing carbostyril derivatives, and more particularly relates to processes and intermediates for preparing aripiprazole and derivatives thereof.

### BACKGROUND OF THE INVENTION

U.S. Patent No. 5,006,528 (Oshiro et al.) discloses a number of novel carbostyril derivatives and methods for their preparation. One of the carbostyril derivatives disclosed by Oshiro et al. is aripiprazole, also known as 2(1H)-Quinolinone, 7-[4-[4-(2,3-dichlorophenyl)-1-piperazinyl]butoxy]-3,4-dihydro-(9Cl), or 7-[4-[4-(2,3-Dichlorophenyl)-1-piperazinyl]butoxy]-3,4-dihydrocarbostyril. Aripiprazole is an atypical antipsychotic agent useful for the treatment of schizophrenia, and has the following structure:

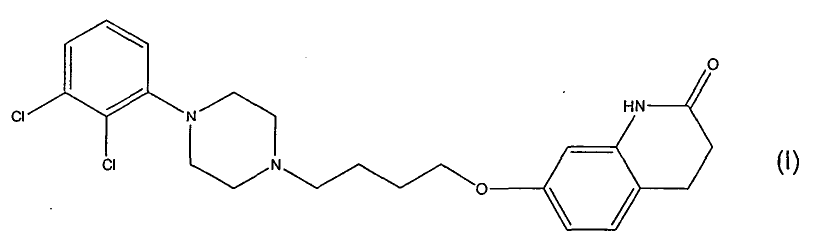

As can be seen from its structure, aripiprazole is comprised of a dichlorophenylpiperazine (DCPP) moiety and a 7-hydroxydihydrocarboxystyryl (HCS) moiety connected by a linking group comprised of four methylene groups. The central location of the linker in the final product, combined with the functional handles on the DCPP (amine) and HCS (phenol) moieties, strongly suggest a stepwise double nucleophilic displacement at the ends of a linker bearing two potential leaving groups. Scheme (I) shows one possible sequence for the double nucleophilic displacement reaction, in which DCPP (II) is first reacted with a linker (IIIa) to form intermediate (V), followed by reaction of intermediate (V) with HCS (IV) to form aripiprazole (I).

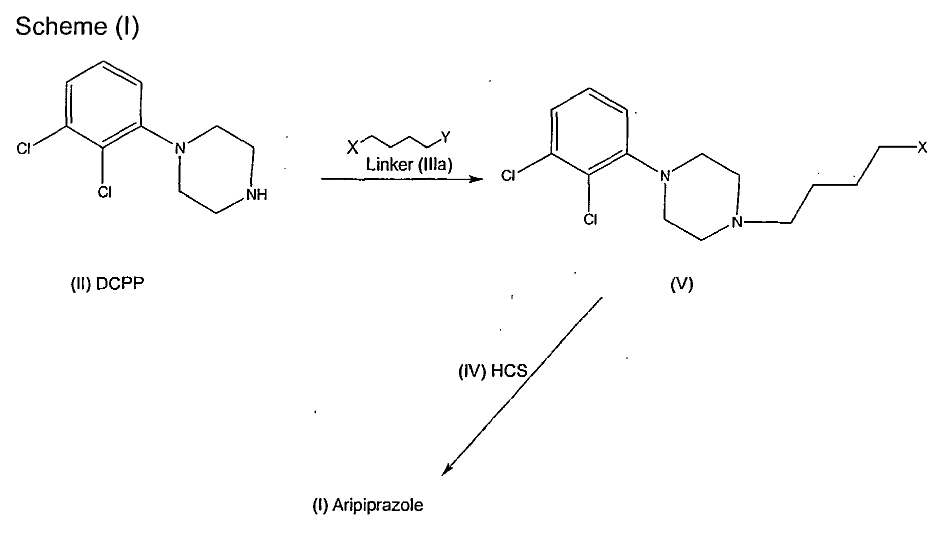

Scheme (II) illustrates another possible sequence for the double nucleophilic displacement reaction, in which the HCS (IV) is first reacted with the linker (IIIa) to form intermediate (VI), followed by reaction of intermediate (VI) with DCPP (II) to form aripiprazole (I).

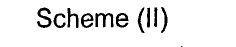

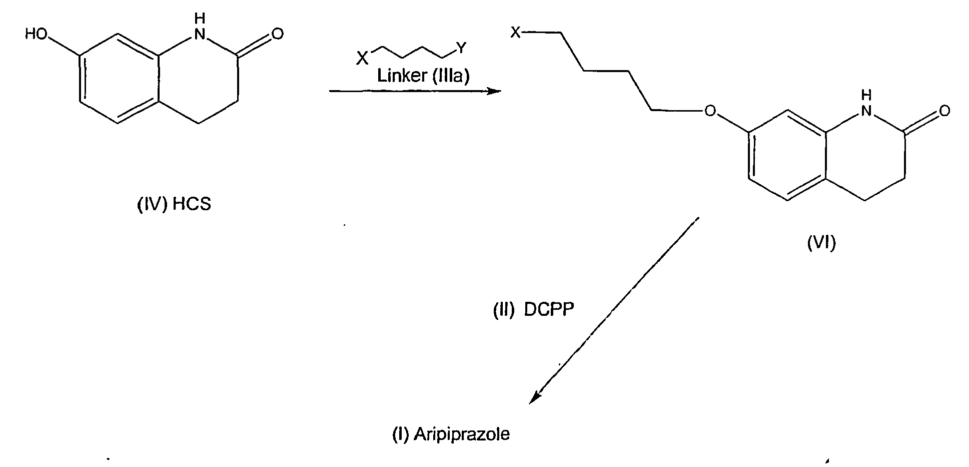

Most of the synthetic routes to aripiprazole and related carbostyril derivatives reported in the literature follow Scheme (II), in which intermediate (VI) is the key intermediate. Scheme (I) and intermediate (V), although mentioned in the Oshiro et al. patent and other publications, have attracted less interest.

The alkylated intermediates (V) and (VI) both have a leaving group X for further substitution, and may lack sufficient stability for easy isolation and purification prior to the final step of the synthesis. Therefore, the need exists for an improved process for preparing aripiprazole and other carbostyril derivatives.

### SUMMARY OF THE INVENTION

The inventors have developed an improved process for producing aripiprazole and other carbostyril derivatives, in which alkylated intermediates (V) and (VI) are avoided. In the process of the invention, shown in Scheme (III) below, DCPP (II) or a salt thereof is reacted with a linker (III) so as to produce a novel quaternary spiro ammonium salt intermediate (VIII), which is an easily isolable and purifiable solid. Intermediate (VIII) is then reacted with HCS (IV) to produce a carbostyril derivative (IX), in which n is either 6 or 8, depending on whether saturated linker (IIIa) or unsaturated linker (IIIb) is used.

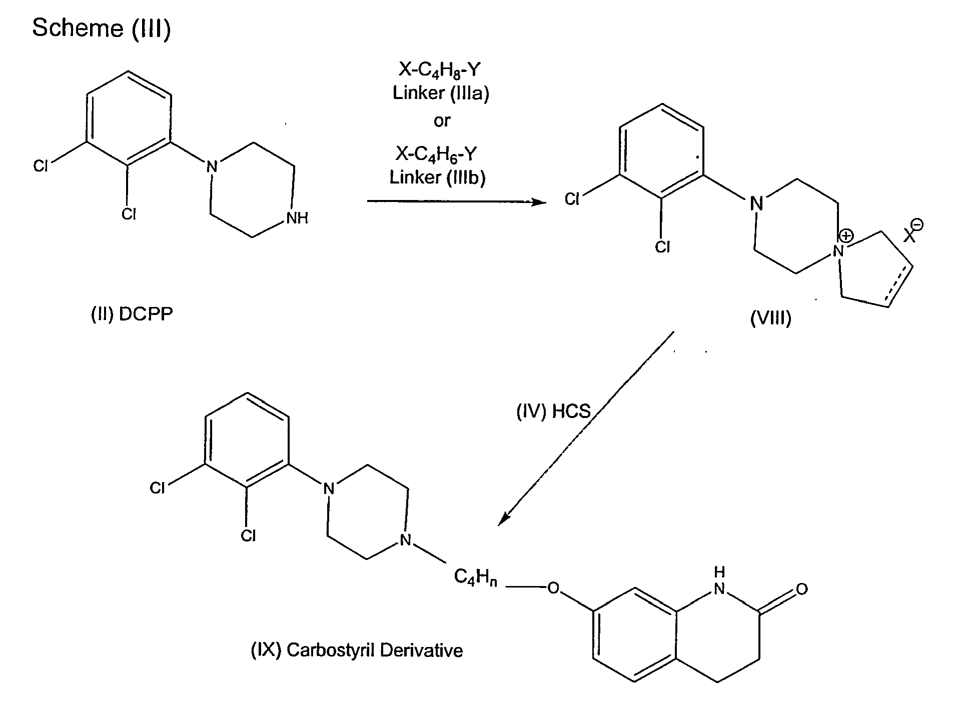

The unsaturated linker (IIIb) results in the formation of an unsaturated 3-pyrrolinyl moiety in the quaternary spiro ammonium salt (VIII), which forms a carbostyril derivative (IX) in which n is 6. On the other hand, the use of the saturated linker (IIIa) results in the formation of a saturated pyrrolidinyl moiety in salt (VIII), which forms a carbostyril derivative (IX) in which n is 8, i.e. aripiprazole (I).

### DETAILED DESCRIPTION OF PREFERRED EMBODIMENTS

The following are detailed descriptions of preferred processes according to the invention for preparing carbostyril derivatives (IX).

As mentioned above, the starting material in the process of the present invention is dichlorophenylpiperazine (DCPP), having formula (II) shown above. DCPP can either be obtained commercially or can be prepared from commercially available reagents. One method for preparing piperazine derivatives such as DCPP is described in Reference Example 1 of the Oshiro et al. patent, and involves the reaction of a substituted aniline with di(2-bromoethyl)amine hydrobromide.

The first step in the process according to the invention comprises reaction of DCPP with linker (III) as described above with reference to Scheme III according to the invention. The leaving groups X and Y of linker (III) may either be the same or different and are preferably selected from the group comprising halogens and sulfonates. Preferred examples of halogens include chlorine, bromine and iodine. Preferred examples of sulfonates include mesylate and tosylate.

In some preferred embodiments, the linker (III) comprises the saturated linker (IIIa), which results in the production of aripiprazole (I). A particularly preferred linker (IIIa) of this type is 1,4-dibromobutane.

In other preferred embodiments, the linker (III) comprises the unsaturated linker (IIIb), and most preferably comprises X-CH₂-CH=CH-CH₂-Y. The use of linker (IIIb) will result in the production of the compound shown below having formula (X), referred to herein as "dehydroaripiprazole", in which the double bond in the linker moiety is preferably cis. A particularly preferred unsaturated linker (IIIb) is 1,4-dichloro-cis-2-butene.

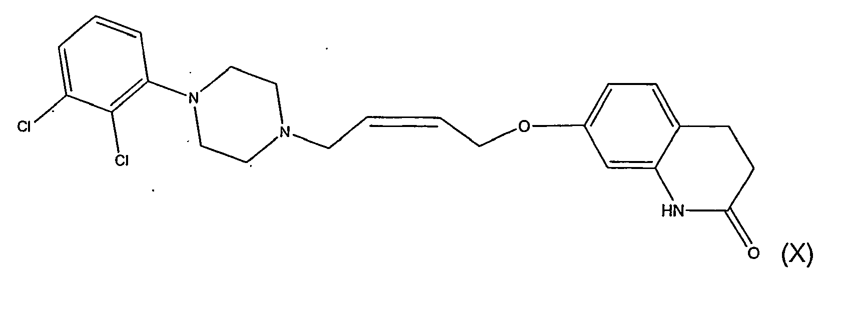

The DCPP (II), in the form of a free base or a hydrochloride, is reacted with linker (III) as shown above in Scheme (III). The reaction is conducted in an organic solvent, in the presence of a base, and is optionally catalyzed by a metal halide. The solvent preferably comprises one or more ketones, with acetone and methyl ethyl ketone (MEK) being particularly preferred. The base can be either organic or inorganic, with metal carbonates or bicarbonates being preferred examples of inorganic bases, and alkali metal carbonates or bicarbonates being particularly preferred. The metal halide catalyst may preferably comprise an alkali metal halide, with alkali metal bromides or iodides being particularly preferred. The reaction is conducted at a temperature of from about 50°C to about 120°C for a period of about 8 to 24 hours, typically 15 hours, depending on the nature of the leaving groups X and Y.

The following are the structures of particularly preferred quaternary spiro ammonium salts (VIII) which can be prepared according to the process of the invention, shown alongside the linkers (III) used in their preparation.

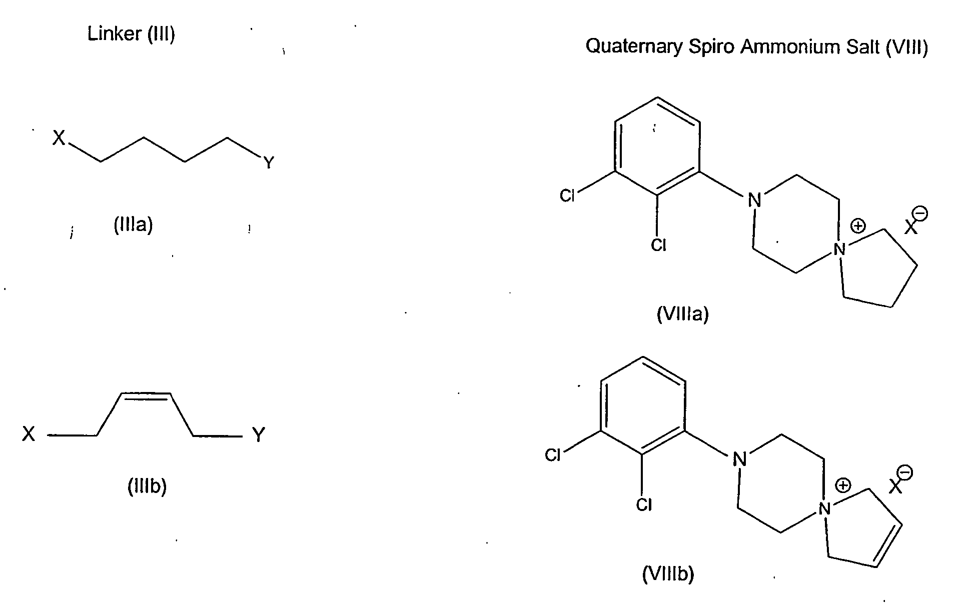

The anion of the quaternary spiro ammonium salt is shown herein as X⁻, an anion having a net charge of -1 which originates from one of the leaving groups of the linker (III). It will be appreciated that the anion of the quaternary spiro ammonium salt (VIII) is not restricted to X⁻ and may be selected from a large group anions having a net charge of -1 or greater.

The quaternary spiro ammonium salts of general formula (VIII) can be isolated by direct filtration, rinsing and drying, and may comprise a mixture of organic and inorganic salts. This mixture can be further purified or can be used directly in the second step of the process.

The second step of the process comprises reaction of the quaternary spiro ammonium salt (VIII) with HCS (IV), thereby producing a carbostyril derivative of formula (IX) as shown in Scheme (III). The HCS is deprotonated by a base, which may be carried over from the first step of the process or which may be added during the second step. Preferably, the base is a carbonate or bicarbonate as in the first step of the process. The HCS is preferably present in the reaction mixture in a stoichiometric or sub-stoichiometric amount relative to the quaternary spiro ammonium salt (VIII), and is typically present in an amount of about 0.95 molar equivalents.

Although many solvents may be suitable for use in the second step of the process, the solvent is preferably selected from one or more members of the group of polar aprotic solvents and ketones. Preferred examples of polar aprotic solvents include dimethyl sulfoxide (DMSO), dimethyl formamide (DMF), 1-methyl-2-pyrrolidinone (NMP), hexamethylphosphoramide (HMPA), and hexamethylphosphorous triamide (HMPT). Preferred ketones include MEK and methyl isobutyl ketone (MIBK). A particularly preferred solvent system comprises a mixture of an aprotic solvent with a ketone, with a mixture of DMF-MIBK being particularly preferred.

Depending at least partly on the solvent system chosen, the reaction temperature of the second step ranges from about 80°C to about 140°C, and is preferably about 120°C. The time of reaction ranges from about 15 to 48 hours, typically about 18 hours.

The carbostyril derivative resulting from the second step can be worked up and purified by known methods, which are further described in the following examples.

Cis-dehydroaripiprazole (X) may be converted to aripiprazole (I) by reduction of the double bond in the linker moiety. The double bond may be reduced by a standard catalytic hydrogenation with palladium on charcoal (50 psi, 20°C, 3h, methanol).

The invention is further illustrated by the following non-limiting examples.

### EXAMPLE 1 - Preparation of 8-(2,3-Dichlorophenyl)-8-aza-5-azoniaspiro[4,5]decane Bromide (VIIIa)

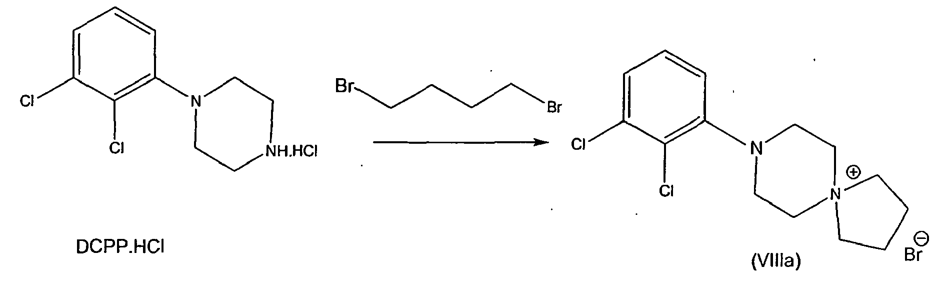

To a suspension of DCPP-HCl (100g) and potassium carbonate (124g) in acetone (700 mL) was added 1,4-dibromobutane (55.8 mL). The resulting mixture was refluxed with vigorous stirring for 15h. After cooling to room temperature, the suspension was filtered. The filter cake was rinsed twice with 100 mL of acetone, and dried under vacuum (35°C, 1 torr) for one hour. The crude quaternary spiro ammonium salt (VIIIa) was obtained as a mixture with inorganic salts (potassium carbonate, bicarbonate, chloride and bromide) in the form of a white powder: 288g. The ratio of organic/inorganic salts was 40/60 (w/w), and therefore the yield was 85%. HPLC at 254 nm (Waters XTerra RP18, 3.5 µM, 3x150 mm, acetonitrile/water (0.1% TFA) - 28/72 (v/v), 0.5 mL/min., retention time: 4.27 min.) detected the quaternary spiro ammonium salt as the sole organic compound.
¹H NMR 300 MHz (D₂O, δ ppm): 6.95 (m, 3H); 3.42 (s, 4H); 3.39 (s, 4H); 3.02 (s, 4H); 2.02 (s, 4H); ¹³C NMR: 148.76; 133.44; 128.61; 126.89; 126.04; 119.96; 63.33; 60.00; 46.67; 21.54.

### EXAMPLE 2 - Purification of 8-(2,3-Dichloro-phenyl)-8-aza-5-azoniaspiro[4,5]decane Bromide (VIIIa)

The crude quaternary spiro ammonium salt (1g) prepared in Example 1 was suspended in isopropyl alcohol (20 mL) and stirred at room temperature for 1 hour. The suspension was filtered and the filtrate was concentrated to a volume of 7 mL. To this solution was added 7 mL of hexanes, and gentle stirring allowed crystallisation of pure quaternary spiro ammonium salt. The salt was filtered and rinsed with a 1/1 mixture of isopropyl alcohol/ hexanes and dried under vacuum, to yield 400 mg of white crystals.

### Example 3 - Preparation of Aripiprazole

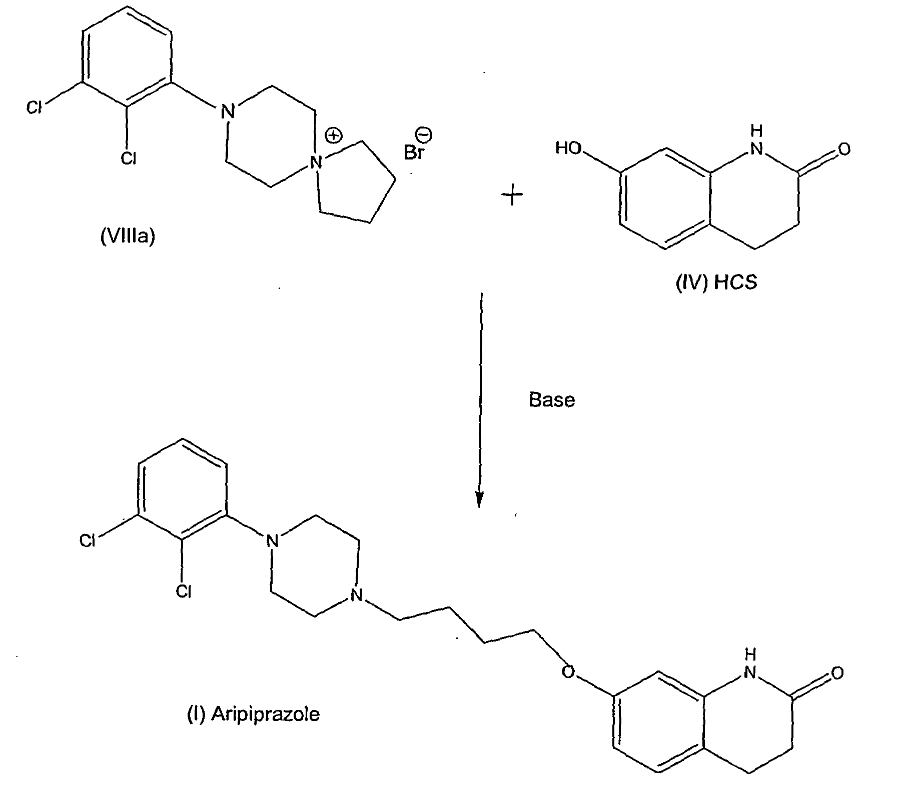

To a suspension of the crude quaternary spiro ammonium salt (VIIIa) of Example 1 (288g) in MIBK (1440 mL) and DMF (215 mL), was added the 7-hydroxy-4,5-dihydrocarbostyryl (HCS) (48.8g). The resulting mixture was refluxed under vigorous stirring for 18h. The reaction temperature was then adjusted in a manner to distil under a water pump vacuum 1150 mL of MIBK, with a column-head temperature ranging from 60 to 70°C. After cooling to room temperature, water (1440 mL) and hexanes (1440 mL) were added, and the suspension stirred for 30 min. The medium was filtered, and the filter cake washed 4 times with 290 mL water and suctioned. The wet filter cake was then recrystallized in ethanol (900 mL), to yield Aripiprazole as a white powder (107.65g). HPLC at 254 nm (Waters XTerra RP18, 3.5 µM, 3x150 mm, acetonitrile/water (0.1% TFA) - 28/72 (v/v), 0.5 mL/min., retention time: 10.55 min.; 99.75%).

Although the invention has been described in connection with certain preferred embodiments, it is not limited thereto. Rather, the invention includes all embodiments which fall within the following claims.

## Claims

1. A process for preparing carbostyril derivatives, comprising:
(a) reacting dichlorophenylpiperazine (DCPP) or an acid addition salt thereof with a compound of formula X-C₄H₈-Y or X-C₄H₆-Y to produce a quaternary spiro ammonium salt having a cation of the general formula 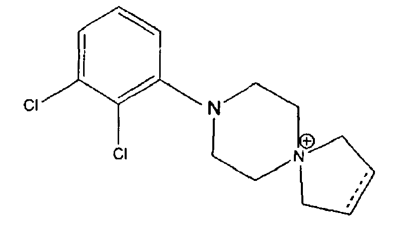 wherein X and Y are leaving groups; and
(b) reacting the quaternary spiro ammonium salt with 7-hydroxydihydrocarboxystyryl (HCS) to produce said carbostyril derivative having the general formula 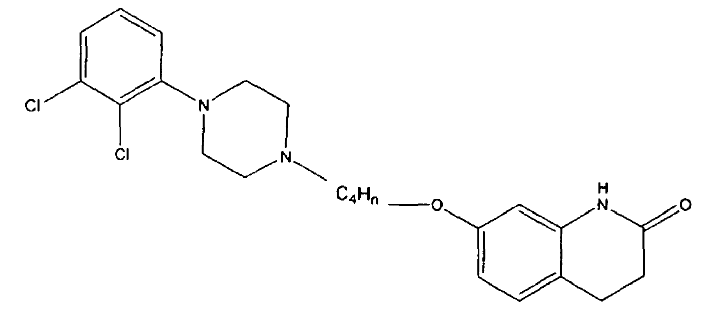
wherein n is 6 or 8.

2. The process according to claim 1, wherein DCPP or an acid addition salt thereof is reacted with a compound of formula X-C₄H₈-Y, wherein n is 8; or is reacted with a compound of formula X-C₄H₆-Y, wherein n is 6.

3. The process according to claim 1, wherein DCPP or an acid addition salt thereof is reacted with a compound of formula X-CH₂-CH=CH-CH₂-Y.

4. The process according to claim 1, wherein the carbostyril derivative is aripiprazole or dehydroaripiprazole.

5. The process according to claim 4, wherein the carbostyril derivative is cis-dehydroaripiprazole.

6. The process according to claim 1, wherein X and Y are each independently selected from the group comprising: halogens such as chlorine, bromine and iodine; and sulfonates such as mesylate and tosylate.

7. The process according to claim 1, wherein the compound reacted with DCPP in step (a) comprises 1,4-dibromobutane or 1,4-dichloro-cis-2-butene.

8. The process according to claim 1, wherein the DCPP is reacted in step (a) as its hydrochloride acid addition salt.

9. A quaternary spiro ammonium salt having a cation comprising

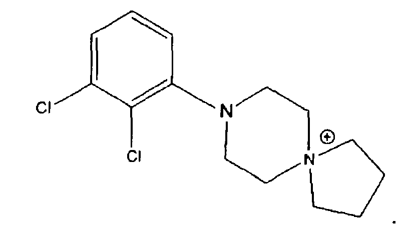

10. A quaternary spiro ammonium salt having a cation comprising

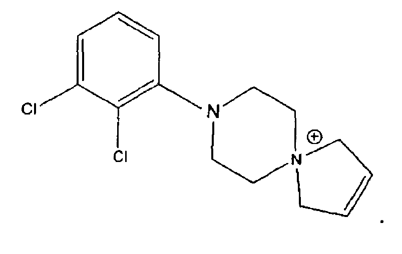

## Patentansprüche

1. Verfahren zur Herstellung von Carbosytrilderivaten, umfassend:
(a) Zur-Reaktion-Bringen von Dichlorphenylpiperazin (DCPP) oder eines sauren Anlagerungssalzes davon mit einer Verbindung der Formel X-C₄H₈-Y oder X-C₄H₆-Y, um ein quartäres Spiro-Ammoniumsalz herzustellen, das ein Kation der allgemeinen Formel 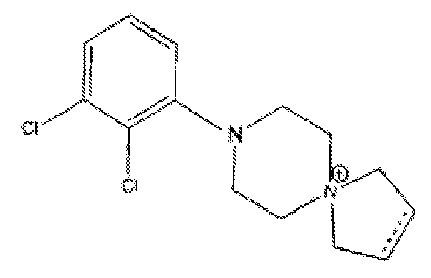 aufweist, in der X und Y Abgangsgruppen sind; und
(b) Zur-Reaktion-Bringen des quartären Spiro-Ammoniumsalzes mit 7-Hydroxydihydrocarboxystyril (HCS) um das Carbostyrilderivat herzustellen, mit der allgemeinen Formel 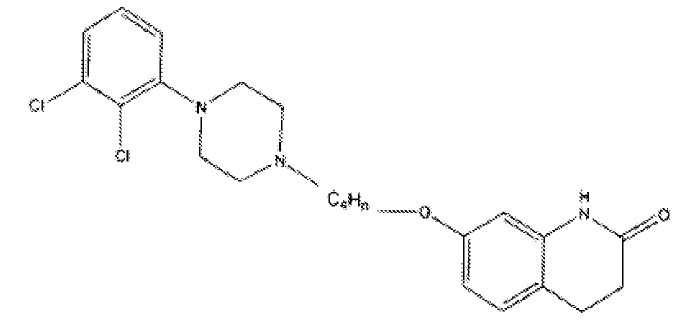
in welcher n 6 oder 8 ist.

2. Verfahren gemäß Anspruch 1, bei dem DCPP oder ein saures Anlagerungssalz davon mit einer Verbindung der Formel X-C₄H₈-Y, in der n 8 ist, zur Reaktion gebracht wird, oder mit einer Verbindung der Formel X-C₄H₆-Y, in der n 6 ist, zur Reaktion gebracht wird.

3. Verfahren gemäß Anspruch 1, bei dem DCPP oder ein saures Anlagerungssalz davon mit einer Verbindung der Formel X-CH₂-CH=CH-CH₂-Y zur Reaktion gebracht wird.

4. Verfahren gemäß Anspruch 1, in dem das Carbostyrilderivat Aripiprazol oder Dehydroaripiprazol ist.

5. Verfahren gemäß Anspruch 4, in dem das Carbostyrilderivat cis-Dehydroaripiprazol ist.

6. Verfahren gemäß Anspruch 1, bei dem X und Y jeweils unabhängig von einander ausgewählt werden aus der Gruppe bestehend aus: Halogenen wie beispielsweise Chlor, Brom und Iod, und Sulfonaten wie beispielsweise Mesylat und Tosylat.

7. Verfahren gemäß Anspruch 1, bei dem die mit DCPP in Schritt (a) zur Reaktion gebrachte Verbindung 1,4- Dibrombutan oder 1,4-Dichlor-cis-2-buten umfasst.

8. Verfahren gemäß Anspruch 1, bei dem das DCPP in Schritt (a) als sein saures Hydrochlorid-Anlagerungssalz zur Reaktion gebracht wird.

9. Quartäres Spiro-Ammoniumsalz mit einem Kation umfassend

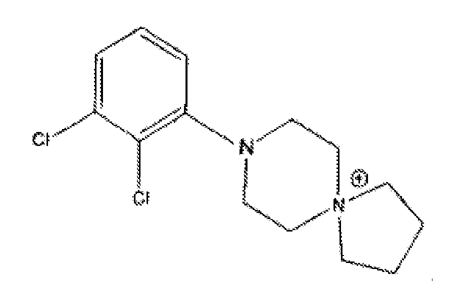

10. Quartäres Spiro-Ammoniumsalz mit einem Kation umfassend

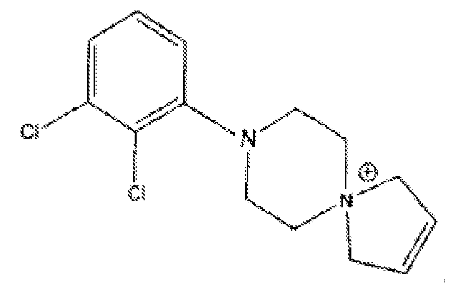

## Revendications

1. Procédé pour préparer des dérivés de carbostyryle, comprenant :
(a) la réaction de dichlorophénylpipérazine (DCPP) ou d'un sel d'addition d'acide de celle-ci avec un composé de formule X-C₄H₈-Y ou X-C₄H₆-Y pour produire un sel de spiro-ammonium quaternaire ayant un cation de formule générale 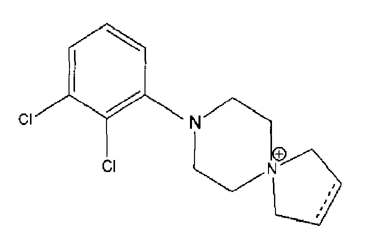 dans laquelle X et Y sont des groupes partants ; et
(b) la réaction du sel de spiro-ammonium quaternaire avec du 7-hydroxydihydrocarboxystyryle (HCS) pour produire ledit dérivé de carbostyryle de formule générale 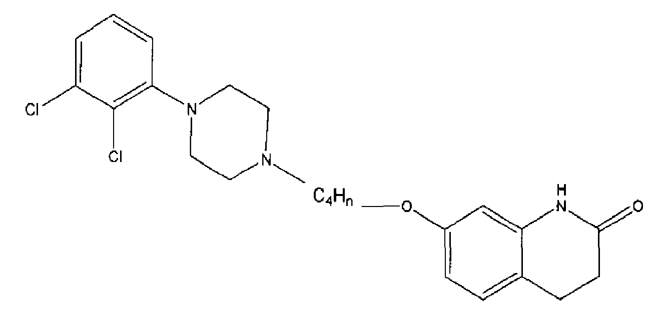
dans laquelle n vaut 6 ou 8.

2. Procédé selon la revendication 1, dans lequel la DCPP ou un sel d'addition d'acide de celle-ci est mis à réagir avec un composé de formule X-C₄H₈-Y, où n vaut 8 ; ou est mis à réagir avec un composé de formule X-C₄H₆-Y, où n vaut 6.

3. Procédé selon la revendication 1, dans lequel la DCPP ou un sel d'addition d'acide de celle-ci est mis à réagir avec un composé de formule X-CH₂-CH=CH-CH₂-Y.

4. Procédé selon la revendication 1, dans lequel le dérivé de carbostyryle est l'aripiprazole ou le déshydroaripiprazole.

5. Procédé selon la revendication 4, dans lequel le dérivé de carbostyryle est le cis-déshydroaripiprazole.

6. Procédé selon la revendication 1, dans lequel chacun de X et Y est indépendamment choisi dans le groupe comprenant : les halogènes tels que le chlore, le brome et l'iode ; et les sulfonates tels que le mésylate et le tosylate.

7. Procédé selon la revendication 1, dans lequel le composé qui réagit avec la DCPP dans l'étape (a) comprend du 1,4-dibromobutane ou du 1,4-dichloro-cis-2-butène.

8. Procédé selon la revendication 1, dans lequel la DCPP est mise à réagir dans l'étape (a) sous la forme de son sel d'addition d'acide chlorhydrique.

9. Sel de spiro-ammonium quaternaire ayant un cation comprenant

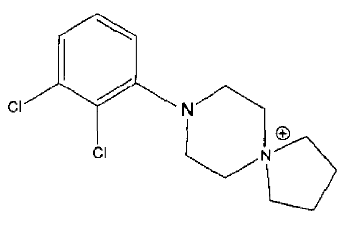

10. Sel de spiro-ammonium quaternaire ayant un cation comprenant
